(19) Europäisches Patentamt
European Patent Office
Office européen des brevets

(11) EP 4 344 698 A1

(12) EUROPEAN PATENT APPLICATION

(43) Date of publication:
03.04.2024 Bulletin 2024/14

(21) Application number: 23175439.1

(22) Date of filing: 25.05.2023

(51) International Patent Classification (IPC):
*A61K 9/00* (2006.01) *A61K 9/08* (2006.01)
*A61K 31/138* (2006.01)

(52) Cooperative Patent Classification (CPC):
A61K 9/08; A61K 9/0053; A61K 9/0095;
A61K 31/138

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.05.2022 GB 202207690

(71) Applicant: Zentiva K.S.
102 37 Praha 10 (CZ)

(72) Inventor: Cernova, Miroslava
169 00 Praha 6 (CZ)

(74) Representative: Ellis, Robin Patrick
Reddie & Grose LLP
The White Chapel Building
10 Whitechapel High Street
London E1 8QS (GB)

Remarks:
Claims 16 to 20 are deemed to be abandoned due to non-payment of the claims fees (Rule 45(3) EPC).

# (54) LIQUID PHARMACEUTICAL FORMULATION OF BISOPROLOL

(57) The present invention relates to liquid pharmaceutical compositions comprising bisoprolol or a pharmaceutically acceptable salt thereof. Further, the invention relates to the use of liquid pharmaceutical compositions comprising bisoprolol or a pharmaceutically acceptable salt thereof in the treatment of heart failure, hypertension and angina pectoris in subjects with a swallowing disorder.

EP 4 344 698 A1

## Description

### Field of the Invention

The present invention relates to liquid pharmaceutical compositions comprising bisoprolol or a pharmaceutically acceptable salt thereof. Further, the invention relates to the use of liquid pharmaceutical compositions comprising bisoprolol or a pharmaceutically acceptable salt thereof in the treatment of heart failure, hypertension and angina pectoris in subjects with a swallowing disorder.

### Background

Bisoprolol ((±)-1-[4-[[2-(1-Methylethoxy)ethoxy]methyl]phenoxy]-3-[(1 methylethyl)amino]-2-propanol)) is a beta blocker medication. The S(-) enantiomer of bisoprolol is mainly responsible for the beta-blocking activity. Beta blockers are a class of medication predominantly used to manage abnormal heart symptoms and to prevent a second heart attack after a first heart attack. They are also widely used to treat high blood pressure. Beta blockers are competitive antagonists that block the receptor sites for the endogenous catecholamines, epinephrine and norepinephrine, on the adrenergic beta receptors of the sympathetic nervous system.

Bisoprolol is cardioprotective. It selectively and competitively blocks catecholamine (adrenaline) stimulation of β1 adrenergic receptors (adrenoreceptors), which are mainly found in the heart muscle cells and heart conduction tissue (cardiospecific) but also found in juxtaglomerular cells in the kidney. Normally, adrenaline and noradrenaline stimulation of the β1 adrenoreceptor activates a signalling cascade (Gs protein and cAMP) which leads to increased contractility and increased heart rate of the heart muscle. By blocking the activation of this cascade bisoprolol decreases the adrenergic stimulation of the heart muscle cells.

An increase in cardiovascular diseases, high blood pressure and hypertension has led to a major increase in the use of beta blockers such as bisoprolol.

Oral administration is the preferred administration route for bisoprolol, which is mainly available in tablet or capsule forms to be administered as a 10 mg maximum daily dosage. However, solid oral dosage forms have several problems.

Manufacturing solid dosage forms often requires complex processes and the finished dosage forms tend to be bulky resulting in higher production and shipping costs.

Orally administered solid dosage forms often take longer to absorb into the blood stream than other dosage forms, rendering them less suitable for medications that need to act quickly.

Orally administered solid dosage forms are inherently unsuitable for subjects with swallowing difficulties which is becoming an increasing problem with an aging population. A reluctance, or difficulty in, swallowing oral dosage forms often leads to compliance issues which, in the long term, results in patients having recurring problems with their condition. Often healthcare practitioners crush tablets in an effort to improve ingestion of the medicament but this may lead to unintentional under- or over-dosing as well as being non-compliant with the administration requirements of the medicament.

Liquid oral dosage forms are a recognised way of addressing these production and compliance issues but they tend to be less stable resulting in a reduced shelf life of the medicament that can affect the efficacy of the API and lead to increased amounts of the medication being thrown away.

Accordingly, there is a need for an oral dosage form that addresses the administration issues associated with solid oral dosage forms, which is also economical to produce and stable.

### Summary of the invention

In a first aspect of the invention, there is provided a liquid pharmaceutical composition comprising bisoprolol or a pharmaceutically acceptable salt thereof having a pH higher than 6.

In a second aspect of the invention there is provided a liquid pharmaceutical composition comprising bisoprolol or a pharmaceutically acceptable salt thereof having a pH higher than 6 for use in the treatment of heart failure, hypertension and angina pectoris in subjects with a swallowing disorder.

Other features, embodiments and advantages will be apparent from the description and the claims.

### Figures

**Figure 1:** A hydrolytic curve showing the change in Impurity A of a solution of bisoprolol fumarate with varying pH stored for 30 days at 60 °C.

## Detailed Description

Liquid pharmaceutical compositions are easier to swallow; provide good dosage flexibility and facilitate the absorption of the active ingredient. They are highly suitable dosage forms for geriatric and mentally disturbed patients and their palatability can be easily varied as per the requirements of the patient through the use of sweeteners, colouring agents and flavouring agents.

The present invention provides alternative dosage forms to solid oral pharmaceutical dosage forms of bisoprolol in the attempt to ease ingestion for patients having a swallowing disorder.

According to the present invention, there is provided a liquid pharmaceutical composition comprising bisoprolol or a pharmaceutically acceptable salt thereof having a pH higher than 6.

Pharmaceutically acceptable salts of bisoprolol may include acetate, aspartate, carbonate, edetate, fumarate, gluconate, glutamate, glycolate, hexanoate, lactate, malate, mesylate, phosphate, propionate, stereate, succinate, sulfate, tartrate, tosylate. Preferably, the pharmaceutically acceptable salt of bisoprolol may be the fumarate salt. As used herein, the terms "fumarate", "hemifumarate" and "monofumarate" may be used interchangeable between each other.

The liquid pharmaceutical composition according to the present invention may comprise the racemate; S-enantiomer or R-enantiomer of bisoprolol or a pharmaceutically acceptable salt thereof. In a preferred embodiment, the liquid pharmaceutical composition according to the invention may comprise the racemate of bisoprolol or a pharmaceutically acceptable salt thereof.

According to the present invention, there is provided a liquid pharmaceutical composition comprising bisoprolol or a pharmaceutically acceptable salt thereof wherein the pH range is from 6 to 9. Preferably, the liquid pharmaceutical composition according to the present invention has a pH of 6.4.

According to the present invention, there is provided a liquid pharmaceutical composition comprising bisoprolol or a pharmaceutically acceptable salt thereof wherein the pH range may be selected from 6.05 to 8.95, from 6.1 to 8.9, from 6.15 to 8.85, from 6.2 to 8.8, from 6.25 to 8.75, from 6.3 to 8.7, from 6.35 to 8.65, from 6.4 to 8.6, from 6.45 to 8.55, from 6.5 to 8.5, from 6.55 to 8.45, from 6.6 to 8.4, from 6.65 to 8.35, from 6.7 to 8.3, from 6.75 to 8.25, from 6.8 to 8.2, from 6.85 to 8.15, from 6.9 to 8.1, from 6.95 to 8.05, from 7 to 8, or combination thereof.

According to the present invention, there is provided a liquid pharmaceutical composition comprising bisoprolol or a pharmaceutically acceptable salt thereof wherein the pH range may be selected from at least 6.05, at least 6.1, at least 6.15, at least 6.2, at least 6.25, at least 6.3, at least 6.35, at least 6.4, at least 6.45, at least 6.5, at least 6.55, at least 6.6, at least 6.65, at least 6.7, at least 6.75, at least 6.8, at least 6.85, at least 6.9, at least 6.95, or at least 7.

According to the present invention, there is provided a liquid pharmaceutical composition comprising bisoprolol or a pharmaceutically acceptable salt thereof wherein the pH range may be selected from no more than 8.95, no more than 8.9, no more than 8.85, no more than 8.8, no more than 8.75, no more than 8.7, no more than 8.65, no more than 8.6, no more than 8.55, no more than 8.5, no more than 8.45, no more than 8.4, no more than 8.35, no more than 8.3, no more than 8.25, no more than 8.2, no more than 8.15, no more than 8.1, no more than 8.05, or no more than 8.

In another aspect of the present invention the liquid pharmaceutical composition comprising bisoprolol or a pharmaceutically acceptable salt thereof may comprise a buffer. As used herein, the term "buffer" defines a solution that can resist pH change upon addition of an acidic or basic component. Every buffer solution has a certain buffer capacity and buffer range. The buffer capacity is the amount of acid or base that can be added before the pH begins to change significantly. The terms "buffer" and "buffer solution" may be used interchangeably throughout the description and claims.

Buffers according to the present invention may be selected from at least one of a phosphate, citrate, citrate-phosphate, maleate, glutamate, acetate, histidine, tromethamine, carbonate, imidazole, "TES" (2-{[1,3-Dihydroxy-2-(hydroxymethyl)propan-2-yl]amino}ethane-1-sulfonic acid), "PIPES" (1,4-Piperazinediethanesulfonic acid), "MOPSO" (β-Hydroxy-4-morpholinepropanesulfonic acid), ethanolamine, "ADA" (2,2',2"-Nitrilotriacetic acid), "ACES" (2-[(2-Amino-2-oxoethyl)amino]ethane-1-sulfonic acid), "BIS-TRIS propane" (2,2'-[Propane-1,3-diylbis(azanediyl)]bis[2-(hydroxymethyl)propane-1,3-diol]), and "TRIS" (tris(hydroxymethyl)aminomethane) buffer.

The buffer according to the present invention may have a concentration selected from 1 mM to 100 mM, 2 mM to 90 mM, 3 mM to 80 mM, 4 mM to 70 mM, 5 mM to 60 mM, 6 mM to 50 mM, 7 mM to 40 mM, 8 mM to 30 mM, or 9 mM to 20 mM. In a preferred embodiment, the liquid pharmaceutical composition according to the invention may comprise a citrate buffer, wherein the citrate buffer may be in a concentration of 30 mM.

In addition to addressing the problems associated with ingesting solid formulations, the present invention provides a stable pharmaceutical liquid dosage form of bisoprolol, that reduces the formation of impurities.

Bisoprolol is known to be susceptible to hydrolytic degradation which leads to the formation of several impurities. (2RS)-1-(4hydroxymethyl-phenoxy)-3-isopropylaminopropan-2-ol (herein referred to as "Impurity A") is the main degradation product.

A list of the most common impurities of bisoprolol is shown in **Table A.**

**Table A:** Most common impurities of bisoprolol.

| Bisoprolol most known impurities | | |
|---|---|---|
| **Chemical structure** | **IUPAC name** | **Common name** |
| 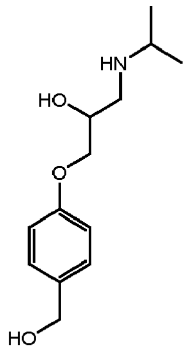 | (2 RS)-1-(4hydroxymethyl-phenoxy)-3-isopropylamino-propan-2-ol | Impurity A |
| 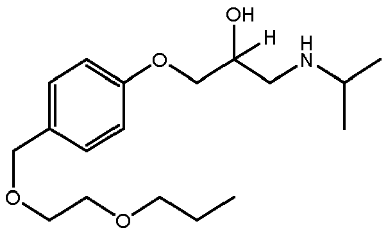 | (2RS)-1-isopropylamino-propan-3-[4-(2-propoxy-ethoxymethyl)-phenoxy]propan-2-ol | Impurity B |
| 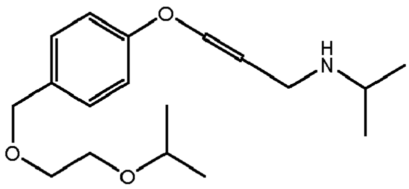 | (EZ)-[3-[4-(2-isopropoxy-ethoxymethyl)phenoxy] allyl]-isopropylamine | Impurity E |
| 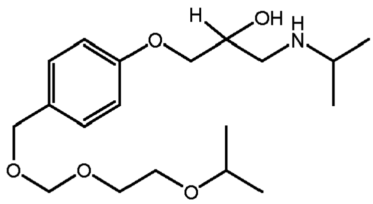 | (2RS)-1-[4-[[(2-isopropoethoxy) methoxy] met yl] phenoxy]-3-isopropylaminopropan-2-ol | Impurity G |
| 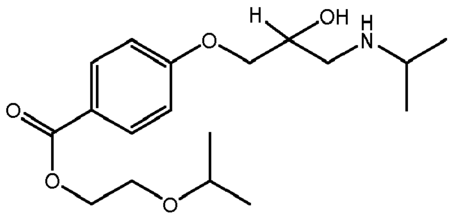 | 2-isopropoxyethyl 4[[(2RS)-2-hydroxy-3-(isopropylamino)propyl]oxy]b enzoate | Impurity K |
| 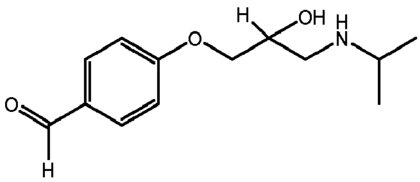 | 4-[[(2RS)-2-hydroxy-3-(isopropylamino)propyl] oxy]-benzaldehyde | Impurity L |

(continued)

| Bisoprolol most known impurities | | |
|---|---|---|
| Chemical structure | IUPAC name | Common name |
| 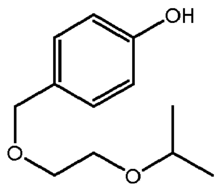 | 4-[(2-Isopropoxyethoxy)methyl] phenol | Impurity M |

The liquid pharmaceutical composition according to the present invention may comprise a sum of impurities in an amount which is less than 0.5%, less than 0.475%, less than 0.45%, less than 0.425%, less than 0.4%, less than 0.375%, less than 0.35%, less than 0.325%, less than 0.3%, less than 0.275%, less than 0.25%, less than 0.225%, less than 0.2%, less than 0.175%, less than 0.150%, less than 0.125%, less than 0.1%, less than 0.075%, less than 0.05%, less than 0.025%. In a further embodiment, the liquid pharmaceutical composition according to the present invention may comprise a sum of impurities in an amount which is less than 0.27%.

Advantageously, it was found that solutions of bisoprolol when kept at a pH above 6 remained stable even at 60 °C after 30 days.

Surprisingly, it has been found that the liquid pharmaceutical compositions according to the present invention also remained stable under stressed condition at 40 °C / 75% RH after 1-month stability testing without the formation of any impurities. Particularly, it has been found that the liquid pharmaceutical composition according to the present invention showed improved stability when compared to the marketed bisoprolol product Concor™COR 1.25 mg film-coated tablets.

Even more surprisingly, it has been found that that the liquid pharmaceutical compositions according to the present invention showed improved stability under stressed condition at 40 °C / 75% RH after 1- and 6-months stability testing when compared to Bisoprolol Fumarate Oral Solution from Rosemont Pharmaceuticals. After 6-months at 40 °C / 75% RH the pharmaceutical composition according to the present invention had less than half of the total impurities compared to the Bisoprolol Fumarate Oral Solution from Rosemont Pharmaceuticals.

In a further aspect of the invention, the liquid pharmaceutical composition comprising bisoprolol or a pharmaceutically acceptable salt thereof may comprise at least one of a co-solvent, an antimicrobial preservative, and a flavouring agent and/or a sweetener.

As used herein, the term "co-solvent" defines a substance added to pharmaceutical formulations to increase the solubility of an active pharmaceutical ingredient (API), thereby increasing its bioavailability. In particular, oral bioavailability depends on several factors including aqueous solubility, drug permeability, dissolution rate, presystemic metabolism, and susceptibility to efflux mechanisms. Co-solvents enhance the bioavailability of oral dosage forms by increasing the solubility of poorly soluble drugs. They work by reducing the interfacial tension between predominately aqueous solution and hydrophobic solutes.

Co-solvents according to the present invention may be selected from at least one of propylene glycol, polyethylene glycol, glycerol, alcohols such as ethanol, methanol or isopropanol, sorbitol, and polyethylene glycols (PEGs) such as PEG-400 or PEG-300. In a preferred embodiment of the invention, the co-solvents is propylene glycol.

According to the present invention, the co-solvent may be from 1% to 30%, from 1.5% to 25%, from 2% to 20% of the liquid pharmaceutical composition. Preferably, the co-solvent may be from 2.5% to 10%, from 3% to 8%, from 3.5% to 7%, from 4% to 6% of the liquid pharmaceutical composition. In a more preferred embodiment of the invention, the liquid pharmaceutical composition may comprise from 4 to 6 % propylene glycol.

As used herein, the term "antimicrobial preservative" defines a chemical substance that is used to preserve pharmaceuticals, food or other organic material from decomposition or fermentation. The mechanism of these chemical compounds ranges from inhibiting growth of bacteria to the inhibition of specific enzymes. Antimicrobial preservatives according to the present invention may be selected from at least one of methyl-, ethyl-, propyl- and butyl-parabens, sorbic acid, sodium-, potassium- and calcium-sorbate, benzoic acid, sodium-, potassium- and calcium-benzoate, sodium meta-bisulfite, benzalkonium chloride, benzyl alcohol, boric acid, borate salts, "BHT" (2,6-Di-tert-butyl-4-methylphenol), "BHA" (butylhydroxyanisole), benzaldehyde, essential oils, and phenol compounds. Preferably the liquid pharmaceutical composition of the invention may comprise methylparaben, propylparaben or a combination of methylparaben and propylparaben.

The antimicrobial preservative may be from 0.01% to 0.5%, from 0.02% to 0.4%, from 0.03% to 0.3%, from 0.04% to 0.2%, from 0.05 to 0.15% of the liquid pharmaceutical composition. In a preferred embodiment of the invention

the liquid pharmaceutical composition may comprise 0.02% to 0.2% of methylparaben and propylparaben. In a more preferred embodiment of the invention, the liquid pharmaceutical composition may comprise 0.18% of methylparaben and 0.02% of propylparaben.

As used herein, the term "flavouring agent" is used to indicate organoleptic excipients which may be added to a pharmaceutical composition to impact aroma, taste, texture, and overall acceptability and palatability. Flavouring agents may include components involving subjective and objective perceptions of taste, feeling factors and odour i.e. aroma, which are important factors in the acceptability and perception of a drug. Flavouring agents are classified as natural or artificial, based on their source. Flavouring agents according to the present invention may be selected from at least one of cherry such as allyl benzoate, pineapple such as allyl caproate, anise such as anisyle alcohol, cinnamon such as cinnamaldehyde, rose such as (2E)-3,7-dimethyl-2,6-octadien-1-ol, peach such as 4-tert-butyl-3-methoxy-2,6-dinitrotoluene, citrus such as D-limonene, raspberry such as 4-(4-hydroxyphenyl)butan-2-one, apricot such as santalyl acetate, strawberry such as 2-methoxynaphthalene, vanilla such as 4-hydroxy-3-methoxybenzaldehyde, and menthol aromas. Preferably the liquid pharmaceutical composition of the invention may comprise raspberry as flavouring agent.

In a further aspect of the invention, the flavouring agent is from 0.05% to 0.5%, from 0.06% to 0.4%, from 0.07% to 0.3%, from 0.08% to 0.2%, from 0.09% to 0.15% of the liquid pharmaceutical composition. In a preferred embodiment of the invention the liquid pharmaceutical composition may comprise 0.13% of a raspberry flavouring agent.

Surprisingly it was found that flavouring agents can have an impact on the stability of the liquid pharmaceutical composition and not all commercially available flavouring agents behave in the same way. While some are completely inert, others lead to an increase in impurities, while others have a stabilizing effect on the liquid pharmaceutical compositions.

According to the present invention, the liquid pharmaceutical composition may not comprise an orange flavouring agent.

As used herein, the term "sweetener" indicates organoleptic excipients which may be added to a pharmaceutical composition to mask unpleasant taste or enhance the perception of sweet taste and to improve palatability in general. Sweeteners may include plant-derived or artificial substances and they may be classified as nutritive and caloric substances or non-nutritive and low-caloric substances. As used herein, nutritive and caloric sweeteners may to some extent be digested in the body and may have food energy value; while non-nutritive and low-caloric sweeteners may not be digested and metabolised by the body and may not have any food energy value. The latter may be suitable sweeteners for diabetic patients.

Nutritive sweeteners according to the present invention may be selected from at least one of glucose, dextrose, fructose, lactose, maltose, sucrose, and tagatose.

Non-nutritive sweeteners according to the present invention may be selected from at least one of acesulfame potassium, maltitol, sorbitol, alitame, aspartame, neotame, saccharin, sucralose, stevia, advantame, sodium cyclamate, sorbitol, xylitol, mannitol, neohesperidin dihydrochalcone, erythritol, lactitol, and thaumatin. Preferably the liquid pharmaceutical composition of the invention may comprise maltitol, more preferably it may comprise sorbitol, even more preferably it may comprise acesulfame potassium.

In a further aspect of the invention, the sweetener is from 0.05% to 50%, from 0.1% to 40%, from 0.15% to 30%, from 0.2% to 20%, from 0.25% to 10% of the liquid pharmaceutical composition. In a preferred embodiment of the invention, the liquid pharmaceutical composition may comprise 40% of maltitol. In a more preferred embodiment, the liquid pharmaceutical composition may comprise 40% of sorbitol. In a most preferred embodiment, the liquid pharmaceutical composition may comprise 0.1% of acesulfame potassium.

The liquid pharmaceutical composition according to the present invention may be for oral administration. Preferably, the liquid pharmaceutical composition of the present invention is a solution or dispersion.

In a further embodiment of the invention the liquid pharmaceutical composition may comprise bisoprolol or a pharmaceutically active salt thereof in a concentration from 0.05 mg/ml to 10 mg/ml, from 0.06 mg/ml to 9 mg/ml, from 0.07 mg/ml to 8 mg/ml, from 0.08 mg/ml to 7 mg/ml, from 0.09 mg/ml to 6 mg/ml, from 0.1 mg/ml to 5 mg/ml, from 0.2 mg/ml to 4 mg/ml, from 0.3 mg/ml to 3 mg/ml, from 0.35 mg/ml to 2 mg/ml, from 0.4 mg/ml to 1.5 mg/ml, from 0.45 mg/ml to 1.25 mg/ml.

The liquid pharmaceutical composition may comprise 0.5 mg/ml of bisoprolol or a pharmaceutically active salt thereof. Alternatively, the liquid pharmaceutical composition may comprise 1.25 mg/ml of bisoprolol or a pharmaceutically active salt thereof.

In a further aspect of the invention, there is provided a liquid pharmaceutical composition comprising bisoprolol or a pharmaceutical accepted salt thereof for use in the treatment of heart failure, hypertension or angina pectoris in subjects with a swallowing disorder.

The subject may be a human subject; male or female. The subject may be above 18 years, above 25 years, above 40 years, above 50 years, above 60 years, above 70 years, above 80 years. Preferably, the subject may be above 65 years old.

Liquid dosage forms for oral use provide the most suitable dosage form for patients who have difficulties in

ingesting tablets or capsules or other solid dosage forms. Swallowing is a complex function involving several nerves and muscles acting in a synchronized reflex mode upon the voluntary swallowing initiation. Swallowing functions can be seriously impacted by diseases and disease progression.

Advantageously, the liquid pharmaceutical composition according to the present invention provides an improved treatment for subjects having swallowing disorders.

Swallowing disorders according to the present invention may arise from at least one of psychological aversion to swallowing, dysphagia, oral cancer, oesophageal cancer, multiple sclerosis, stroke, brain injury, spinal cord injury, Parkinson's disease, amyotrophic lateral sclerosis, muscular dystrophy, cerebral palsy, Alzheimer's disease, head or neck injuries, mouth or neck surgery, bad teeth, missing teeth and ill-fitting dentures.

The liquid pharmaceutical compositions according to the present invention may form part of a long-term treatment regimen, which may require regular monitoring by the doctor. As used herein, the term "dose" defines the specific amount of medication in millilitres (ml) or milligrams (mg) administered to a subject at a point in time. As used herein, the term "dosage regimen" defines the number of doses administered to a subject over a fixed period of time. As used herein, the term "daily dosage" defines the sum of the doses administered in a 24 hour period. The dosage regime is often calculated on the basis of a patient's weight. Treatment with bisoprolol may require a gradual increase of the daily dosage. In contrast to solid dosage forms which are only available as fixed doses, the liquid pharmaceutical composition according to the present invention may be administered in varying doses according to the requirements of the subject.

The liquid pharmaceutical composition according to the invention may be administered in a daily dosage selected from 1 ml to 20 ml, 1.5 ml to 19 ml, 2 ml to 18 ml, 2.5 ml to 17 ml, 3 ml to 16 ml, 3.5 ml to 15 ml, 4 ml to 14 ml, 4.5 ml to 13 ml, 5 ml to 12 ml, 5.5 ml to 11 ml, 6 ml to 10 ml, 6.5 ml to 9 ml, 7 ml to 8 ml. The daily dosage may be 20 ml. Alternatively the daily dosage may be 8 ml. The daily dosage may be administered as one dose or may be provided in two, three, four or five doses. By way of example, the daily dosage may be 8 ml administered as two doses.

The daily dosage may comprise 1 mg to 15 mg, 2 mg to 14 mg, 3 mg to 13 mg, 4 mg to 12 mg, 5 mg to 11 mg, 6 mg to 10 mg of bisoprolol or a pharmaceutically acceptable salt thereof. Preferably the maximum daily dosage of bisoprolol or a pharmaceutically acceptable salt thereof is 10 mg.

Preferably, the dose and/or daily dosage may be administered with food.

In a preferred embodiment of the present invention, the liquid pharmaceutical composition may comprise:

a) from 0.05 mg/ml to 10 mg/ml of bisoprolol or a pharmaceutically acceptable salt thereof;
b) from 1% to 30% of co-solvent;
c) from 0.01% to 0.5% of antimicrobial preservative;
d) from 0.05% to 0.5% of flavouring agent and/or from 0.05% to 50% of sweetener;
e) a 1 mM to 100 mM buffer solution up to 100% of the pharmaceutical composition.

The present invention is now described in more details by, but not limited to, the following Examples.

## Examples

*Example 1 - Preparation of solutions of bisoprolol with differing pH values*

Five different aqueous bisoprolol solutions with a pH ranging from 5 to 9 were prepared by mixing:

- bisoprolol hemifumarate in a concentration of 1.25 mg/ml;
- buffer solution in concentration 10 mmol/l.

Each solution contained a different aqueous buffer solution according to the desired pH:

- a formate buffer was used for pH 5,
- a phosphate buffer was used for pHs 6.2, 7, and 8,
- a borate buffer was used for pH 9.

*Example 2 - Stability testing to examine the effect of pH and temperature on the solutions of Example 1*

In order to examine the effect of pH and temperature on the stability of bisoprolol the solutions prepared according to Example 1 were stored for 30 days at 25 °C, 40 °C and 60 °C.

**Table 1:** Results of stability after 30 days at 25 °C.

| **Bisoprolol in different pHs - 30 days at 25°C** | | |
|---|---|---|
| **pH** | Sum of Impurities (%) | Imp A (%) |
| **5** | 0 | Nd |
| **6.2** | 0 | Nd |
| **7** | 0 | Nd |
| **8** | 0 | Nd |
| **9** | 0 | Nd |
| Nd = impurity was not detected | | |

**Table 2:** Results of stability after 30 days at 40 °C.

| **Bisoprolol in different pHs - 30 days at 40°C** | | |
|---|---|---|
| **pH** | Sum of Impurities (%) | Imp A (%) |
| **5** | 0.15 | 0.15 |
| **6.2** | 0 | Nd |
| **7** | 0 | Nd |
| **8** | 0 | Nd |
| **9** | 0 | Nd |
| Nd = impurity was not detected | | |

**Table 3:** Results of stability after 30 days at 60 °C.

| **Bisoprolol in different pHs - 30 days at 60°C** | | |
|---|---|---|
| **pH** | Sum of Impurities (%) | Imp A (%) |
| **5** | 1.65 | 1.65 |
| **6.2** | 0.14 | 0.14 |
| **7** | 0.04 | 0.04 |
| **8** | 0 | Nd |
| **9** | 0 | Nd |
| Nd = impurity was not detected | | |

**Tables 1, 2 and 3** show that even under stressed conditions at 40 °C and 60 °C, the compositions remain stable for 30 days at pH levels of 6 and above. This trend is also shown in **Figure 1.**

*Example 3 - Preparation of liquid pharmaceutical compositions of bisoprolol*

Liquid pharmaceutical composition A was prepared by dissolving the following components in an aqueous 50 mM phosphate buffer solution at pH 7:

- bisoprolol hemifumarate in a concentration of 1.25 mg/ml;
- propylene glycol in an amount equal to 5.0% of the liquid composition;
- methylparaben in an amount equal to 0.1% of the liquid composition;
- menthol in an amount equal to 0.01% of the liquid composition.

Liquid pharmaceutical composition B was prepared by dissolving the following components in an aqueous 10 mM citrate buffer solution at pH 6.5:

- bisoprolol hemifumarate in a concentration of 1.25 mg/ml;
- propylene glycol in an amount equal to 5.0% of the liquid composition;
- methylparaben in an amount equal to 0.1% of the liquid composition;
- menthol in an amount equal to 0.01% of the liquid composition.

Liquid pharmaceutical composition C was prepared by dissolving the following components in an aqueous 10 mM citrate buffer at pH 6.5:

- bisoprolol hemifumarate in a concentration of 0.5 mg/ml;
- propylene glycol in an amount equal to 5.0% of the liquid composition;
- methylparaben in an amount equal to 0.18% of the liquid composition;
- propylparaben in an amount equal to 0.02% of the liquid composition;
- raspberry aroma in an amount equal to 0.13% of the liquid composition.

Liquid pharmaceutical composition D was prepared by dissolving the following components in an aqueous 30 mM citrate buffer at pH 6.4:

- bisoprolol hemifumarate in a concentration of 0.5 mg/ml;
- propylene glycol in an amount equal to 5.0% of the liquid composition;
- methylparaben in an amount equal to 0.18% of the liquid composition;
- propylparaben in an amount equal to 0.02% of the liquid composition;
- acesulfame potassium in an amount equal to 0.10% of the liquid composition.

Reference composition consisting of the marketed drug product Bisoprolol Fumarate Oral Solution from Rosemont Pharmaceuticals:

- bisoprolol hemifumarate in a concentration of 0.5 mg/ml;
- propylene glycol in an amount equal to 4.0% of the liquid composition;
- ethylparaben in an amount equal to 0.7% of the liquid composition;
- acesulfame potassium in an amount equal to 0.10% of the liquid composition;
- aqueous 1 mM citrate - phosphate buffer at pH 6.3.

Liquid pharmaceutical compositions A, B, C and D and the reference composition are provided in **Table 4.**

**Table 4:** Liquid pharmaceutical compositions A, B, C and D.

| **Component name** | **Sample A** | **Sample B** | **Sample C** | **Sample D** | **Reference composition** |
|---|---|---|---|---|---|
| **Bisoprolol fumarate** | 1.25 mg/ml | 1.25 mg/ml | 0.5 mg/ml | 0.5 mg/ml | 0.5 mg/ml |
| **Propylene glycol** | 5.0% | 5.0% | 5.0% | 5.0% | 4.0% |
| **Methylparaben** | 0.1% | 0.1% | 0.18% | 0.18% | - |
| **Propylparaben** | - | - | 0.02% | 0.02% | - |
| **Ethylparaben** | - | - | - | - | 0.7% |
| **Menthol** | 0.01% | 0.01% | - | - | - |
| **Raspberry aroma** | - | - | 0.13% | - | - |
| **Acesulfame potassium** | - | - | | 0.10% | 0.10% |
| **50mM Phosphate buffer pH 7.0** | ad 100.0% | - | - | - | - |
| **10mM Citrate buffer pH 6.5** | - | ad 100.0% | ad 100.0% | - | - |
| **10mM Citrate buffer pH 6.4** | - | - | - | ad 100.0% | - |

(continued)

| Component name | Sample A | Sample B | Sample C | Sample D | Reference composition |
|---|---|---|---|---|---|
| **1 mM Citrate-Phosphate buffer pH 6.3** | - | - | - | - | ad 100.0% |

*Example 4 - Stability studies comparing compositions A and B with Concor™COR*

Compositions A and B were prepared according to Example 3.

A stability test at 40 °C / 75% RH for 1-month was performed.

Concor™COR 1.25 mg film-coated tablets (batch number 455317) packed in original packaging OPA/Alu/PVC/Alu blister was used.

On conclusion of the stability tests, the impurities in compositions A, B and Concor™COR were analysed by means of HPLC and are provided in **Table 5, 6, 7.**

**Table 5:** Results of 1-month stability of A in regime 40 °C / 75% RH.

| Composition A | | | | | | |
|---|---|---|---|---|---|---|
| **Regime** | **Sum of Imps(%)** | **Imp A** | **Imp L** | **Imp G** | **Imp K** | **Imp M** |
| **Unstressed** | <0.05 | <0.05 | <0.05 | <0.05 | <0.05 | <0.05 |
| **1-month 40°C/75RH** | <0.05 | <0.05 | <0.05 | <0.05 | <0.05 | <0.05 |

**Table 6:** Results of 1-month stability of B in regime 40 °C / 75% RH.

| Composition B | | | | | | |
|---|---|---|---|---|---|---|
| **Regime** | **Sum of Imps(%)** | **Imp A** | **Imp L** | **Imp G** | **Imp K** | **Imp M** |
| **Unstressed** | <0.05 | <0.05 | <0.05 | <0.05 | <0.05 | <0.05 |
| **1-month 40°C/75RH** | <0.05 | <0.05 | <0.05 | <0.05 | <0.05 | <0.05 |

**Table 7:** Results of 1-month stability of Concor™COR 1.25 mg film-coated tablets in regime 40 °C / 75% RH.

| Concor™ COR 1.25 mg film-coated tablets | | | | | | |
|---|---|---|---|---|---|---|
| **Regime** | **Sum of Imps(%)** | **Imp A** | **Imp L** | **Imp G** | **Imp K** | **Imp M** |
| **Unstressed** | 0.98 | <0.05 | 0.15 | 0.05 | 0.12 | 0.16 |
| **1-month 40°C/75RH** | 1.66 | 0.07 | 0.2 | 0.07 | 0.23 | 0.34 |

Two different HPLC methods were used for the quantitative and qualitative determination of impurities.

1 METHOD

Samples as prepared in Example 3 were directly injected into the column.

**Separation conditions**

| | |
|---|---|
| Column: | XSelect CSH Phenyl-Hexyl, 2.5 μm, 100 x 4.6 mm (Waters) |
| Mobile phase: | Component A with component B according to the gradient program |

(continued)

| | |
|---|---|
| Component A: | Carbonate buffer pH 10.8<br>To be prepared by weighting 1.06 g of sodium carbonate anhydrous and 0.17 g of sodium hydrogen carbonate into 1000 ml volumetric flask. Fill it with water for chromatography up to the 1000 ml mark and mix it properly. Then adjust the pH at the value 11.00 ± 0.05 with 1M NaOH. |
| Component B: | Acetonitrile |
| Flow-rate: | 1.0 ml/min |
| Injection volume: | 20.0 μl |
| Autosampler temperature: | 5 °C |
| Column temperature: | 40 °C |
| Detection: | UV detector set up at the wavelength of 225 nm for bisoprolol fumarate and its impurities, except for impurity K<br>UV detector set up at the wavelength of 257 nm for impurity K of bisoprolol fumarate<br>Sampling rate of 2 points/s |

**Gradient program**

| Time [min] | Component A [%] | Component B [%] |
|---|---|---|
| 0.0 | 95 | 5 |
| 1.5 | 95 | 5 |
| 3.0 | 80 | 20 |
| 6.0 | 80 | 20 |
| 13.5 | 20 | 80 |
| 14.5 | 20 | 80 |
| 15.0 | 95 | 5 |
| 18.0 | 95 | 5 |

**Table of peaks**

| | Approximate retention time [min] | Correction factor |
|---|---|---|
| Fumaric acid (RRT 0.09) | 1.0 | - |
| Imp M (RRT 0.47) | 5.1 | - |
| Imp A (RRT 0.57) | 6.2 | - |
| Imp L (RRT 0.81) | 8.7 | - |
| Bisoprolol | 10.8 | - |
| Imp K (RRT 1.02) | 11.0 | 0.5 |

2 METHOD: ANALYSIS OF Concor™COR 1.25 mg film-coated tablets

The sample of Concor™COR 1.25 mg film-coated tablets was prepared by weighing an amount corresponding

to 8 tablets into a 20 ml volumetric flask. The flask was filled up to 20 ml with a solvent mixture of 20% acetonitrile/ 80% water. The solution was sonicated with an ultrasonic bath for 30 minutes and then stirred for 20 minutes. The remaining undissolved material was removed by centrifugation (10 mini 14*$10^3$ rpm). In case some extra particles remained even after the centrifugation, the undissolved solid was removed with filtration by using a Fisher PTFE syringe filters 0.45 μm.

**Separation conditions**

| | |
|---|---|
| Column: | ACE C18 5 μm, 250 x 4.6 mm or equivalent |
| Mobile phase: | Component A with component B according to the gradient program |
| Component A: | Phosphoric acid R 5.9 ml in 1000 ml of water for chromatography R |
| Component B: | Phosphoric acid R 5.9 ml in 1000 ml of acetonitrile R |
| Flow-rate: | 1.0 ml/min |
| Needle wash: | Water and acetonitrile 50 + 50, v/v |
| Injection volume: | 10 μl |
| Autosampler temperature: | 20 °C |
| Column temperature: | 20 °C |
| Detection: | UV detector set up at the wavelength of 225 nm for bisoprolol fumarate and its impurities, except for impurity K<br>UV detector set up at the wavelength of 257 nm for impurity K of bisoprolol fumarate<br>Sampling rate of 2 points/s |

**Gradient program**

| Time [min] | Component A [%] | Component B [%] |
|---|---|---|
| 0.0 | 95 | 5 |
| 4.0 | 95 | 5 |
| 8.0 | 80 | 20 |
| 15.0 | 80 | 20 |
| 34.0 | 20 | 80 |
| 36.0 | 20 | 80 |
| 37.0 | 95 | 5 |
| 45.0 | 95 | 5 |

**Table of peaks**

| | Approximate retention time [min] | Correction factor |
|---|---|---|
| Fumaric acid (RRT 0.25) | 5.3 | - |
| Imp A (RRT 0.49) | 10.0 | 0.7 |
| Imp L (RRT 0.55) | 11.5 | - |
| Imp R (RRT 0.93) | 19.9 | - |
| Bisoprolol | 21.5 | - |

(continued)

| | Approximate retention time [min] | Correction factor |
|---|---|---|
| Imp G (RRT 1.03) | 22.1 | - |
| Imp K (RRT 1.05) | 22.5 | 0.5 |
| Imp B (RRT 1.08) | 22.8 | - |
| Imp E (RRT 1.10) | 23.6 | - |

The results obtained by both HPLC methods where used to calculate the percentage of each individual impurity of bisoprolol fumarate (*x*) using the following equation:

$$x = \frac{A_{SB} * W_{RB} * M * 0.1}{A_{RB} * W_{SB} * D}$$

$A_{SB}$ Peak area of bisoprolol in the chromatogram of the sample solution
$A_{RB}$ Peak area of bisoprolol in the chromatogram of the reference solution
$W_{RB}$ Weight of bisoprolol fumarate RS in mg, recalculated with respect to the potency
$W_{SB}$ Weight of sample in mg
M Average mass of 1 tablet in mg
D Declared amount of bisoprolol fumarate in 1 tablet in mg (1.25 mg).
0.1 Dilution.

*Example 5 - Stability studies comparing compositions* C *and* D *with the Bisoprolol Fumarate Oral Solution from Rosemont Pharmaceuticals*

Compositions C and D were prepared according to Example 3.
A stability test at 40 °C / 75 RH for 6-months was performed with the level of impurities being measured at 1- and 6- months.
The outcome of the stability test on compositions C, D and Bisoprolol Fumarate Oral Solution from Rosemont Pharmaceuticals are provided in **Tables 8, 9, 10.**

**Table 8:** Results of 1-, 6-months stability of C.

| **Composition C** | | | | | | |
|---|---|---|---|---|---|---|
| **Regime** | **Sum of Imps(%)** | **Imp A** | **Imp L** | **Imp G** | **Imp K** | **Imp M** |
| **Unstressed** | <0.05 | <0.05 | <0.05 | <0.05 | <0.05 | <0.05 |
| **1-month 40°C/75RH** | <0.05 | <0.05 | <0.05 | <0.05 | <0.05 | <0.05 |
| **6-month 40°C/75RH** | 0.11 | 0.11 | <0.05 | <0.05 | <0.05 | <0.05 |

**Table 9:** Results of 1-, 6-months stability of D.

| **Composition D** | | | | | | |
|---|---|---|---|---|---|---|
| **Regime** | **Sum of Imps(%)** | **Imp A** | **Imp L** | **Imp G** | **Imp K** | **Imp** M |
| **Unstressed** | <0.05 | <0.05 | <0.05 | <0.05 | <0.05 | <0.05 |
| **1-month 40°C/75RH** | <0.05 | <0.05 | <0.05 | <0.05 | <0.05 | <0.05 |
| **6-month 40°C/75RH** | 0.06 | 0.06 | <0.05 | <0.05 | <0.05 | <0.05 |

**Table 10:** Results of 1-, 6-months stability of reference composition.

| Reference composition | | | | | | |
|---|---|---|---|---|---|---|
| **Regime** | **Sum of Imps(%)** | **Imp A** | **Imp L** | **Imp G** | **Imp K** | **Imp M** |
| **Unstressed** | 0.06 | <0.05 | <0.05 | <0.05 | <0.05 | <0.05 |
| **1-month 40°C/75RH** | 0.08 | <0.05 | <0.05 | <0.05 | <0.05 | <0.05 |
| **6-month 40°C/75RH** | 0.27 | <0.05 | 0.05 | <0.05 | <0.05 | <0.05 |

## Claims

1. A liquid pharmaceutical composition comprising bisoprolol or a pharmaceutically acceptable salt thereof having a pH higher than 6.

2. The pharmaceutical composition according to claim 1 wherein the pH range is from 6 to 9.

3. The pharmaceutical composition according to any of the preceding claims comprising a buffer.

4. The pharmaceutical composition according to claim 3 wherein the buffer is selected from at least one of phosphate, citrate, citrate-phosphate, maleate, glutamate, acetate, histidine, tromethamine, carbonate, imidazole, TES, PIPES, MOPSO, ethanolamine, ADA, ACES, BIS-TRIS propane, and TRIS buffer.

5. The pharmaceutical composition according to claims 3 and 4 wherein the buffer has a concentration selected from 1 mM to 100 mM, 2 mM to 90 mM, 3 mM to 80 mM, 4 mM to 70 mM, 5 mM to 60 mM, 6 mM to 50 mM, 7 mM to 40 mM, 8 mM to 30 mM, or 9 mM to 20 mM.

6. The pharmaceutical composition according to any preceding claims comprising a sum of impurities in an amount which is less than 0.5%, less than 0.475%, less than 0.45%, less than 0.425%, less than 0.4%, less than 0.375%, less than 0.35%, less than 0.325%, less than 0.3%, less than 0.275%, less than 0.25%, less than 0.225%, less than 0.2%, less than 0.175%, less than 0.150%, less than 0.125%, less than 0.1%, less than 0.075%, less than 0.05%, less than 0.025%.

7. The pharmaceutical composition according to any one of the preceding claims further comprising at least one of a co-solvent, an antimicrobial preservative, and a flavouring agent and/or a sweetener.

8. The pharmaceutical composition according to claim 7 wherein the co-solvent is selected from at least one of propylene glycol, polyethylene glycol, glycerol, alcohols such as ethanol, methanol or isopropanol, sorbitol, and polyethylene glycols (PEGs) such as PEG-400 or PEG-300.

9. The pharmaceutical composition according to claims 7 and 8 wherein the co-solvent is from 1% to 30%, from 1.5% to 25%, from 2% to 20%, from 2.5% to 10%, from 3% to 8%, from 3.5% to 7%, from 4% to 6% of the liquid pharmaceutical composition.

10. The pharmaceutical composition according to claims 7 to 9 wherein the antimicrobial preservative is selected from at least one of methyl-, ethyl-, propyl- and butyl-parabens, sorbic acid, sodium-, potassium- and calcium-sorbate, benzoic acid, sodium-, potassium- and calcium-benzoate, sodium meta-bisulfite, benzalkonium chloride, benzyl alcohol, boric acid, borate salts, BHT, BHA, benzaldehyde, essential oils, and phenol compounds.

11. The pharmaceutical composition according to claims 7 to 10 wherein the antimicrobial preservative is 0.01% to 0.5%, from 0.02% to 0.4%, from 0.03% to 0.3%, from 0.04% to 0.2%, from 0.05 to 0.15% of the liquid pharmaceutical composition.

12. The pharmaceutical composition according to claims 7 to 11 wherein the flavouring agent is selected from at least one of cherry, pineapple, anise, cinnamon, rose, peach, citrus, raspberry, apricot, strawberry, vanilla, and menthol aromas.

**13.** The pharmaceutical composition according to claims 7 to 12 wherein the flavouring agent is from 0.05% to 0.5%, from 0.06% to 0.4%, from 0.07% to 0.3%, from 0.08% to 0.2%, from 0.09% to 0.15% of the liquid pharmaceutical composition.

**14.** The pharmaceutical composition according to claims 7 to 13 wherein the sweetener is selected from at least one of glucose, dextrose, fructose, lactose, maltose, sucrose, tagatose, acesulfame potassium, maltitol, sorbitol, alitame, aspartame, neotame, saccharin, sucralose, stevia, advantame, sodium cyclamate, sorbitol, xylitol, mannitol, neohesperidin dihydrochalcone, erythritol, lactitol, and thaumatin.

**15.** The pharmaceutical composition according to claims 7 to 14 wherein the sweetener is from 0.05% to 50%, from 0.1% to 40%, from 0.15% to 30%, from 0.2% to 20%, from 0.25% to 10% of the liquid pharmaceutical composition.

**16.** The pharmaceutical composition according to any preceding claim wherein the bisoprolol or a pharmaceutically active salt thereof is in a concentration from 0.05 mg/ml to 10 mg/ml, from 0.06 mg/ml to 9 mg/ml, from 0.07 mg/ml to 8 mg/ml, from 0.08 mg/ml to 7 mg/ml, from 0.09 mg/ml to 6 mg/ml, from 0.1 mg/ml to 5 mg/ml, from 0.2 mg/ml to 4 mg/ml, from 0.3 mg/ml to 3 mg/ml, from 0.35 mg/ml to 2 mg/ml, from 0.4 mg/ml to 1.5 mg/ml, from 0.45 mg/ml to 1.25 mg/ml.

**17.** A pharmaceutical composition according to any preceding claim for use in the treatment of heart failure, hypertension or angina pectoris in subjects with a swallowing disorder.

**18.** The pharmaceutical composition for use in the treatment according to claim 17 wherein the swallowing disorder arises from at least one of a psychological aversion to swallowing, dysphagia, oral cancer, oesophageal cancer, multiple sclerosis, stroke, brain injury, spinal cord injury, Parkinson's disease, amyotrophic lateral sclerosis, muscular dystrophy, cerebral palsy, Alzheimer's disease, head or neck injuries, mouth or neck surgery, bad teeth, missing teeth and ill-fitting dentures.

**19.** The pharmaceutical composition for use in the treatment according to claims 17 or 18 wherein the subject is at least 65 years old.

**20.** The pharmaceutical composition for use in the treatment according to claims 17 to 19 wherein the composition is administered in a daily dosage selected from 1 ml to 20 ml, from 1.5 ml to 19 ml, from 2 ml to 18 ml, from 2.5 ml to 17 ml, from 3 ml to 16 ml, 3.5 ml to 15 ml, 4 ml to 14 ml, 4.5 ml to 13 ml, 5 ml to 12 ml, 5.5 ml to 11 ml, 6 ml to 10 ml, 6.5 ml to 9 ml, and 7 ml to 8 ml.

**21.** A liquid pharmaceutical composition comprising:

a) from 0.05 mg/ml to 10 mg/ml of bisoprolol or a pharmaceutically acceptable salt thereof;
b) from 1% to 30% of co-solvent;
c) from 0.01% to 0.5% of antimicrobial preservative;
d) from 0.05% to 0.5% of flavouring agent and/or from 0.05% to 50% of sweetener;
e) a 1 mM to 100 mM buffer solution up to 100% of the pharmaceutical composition.

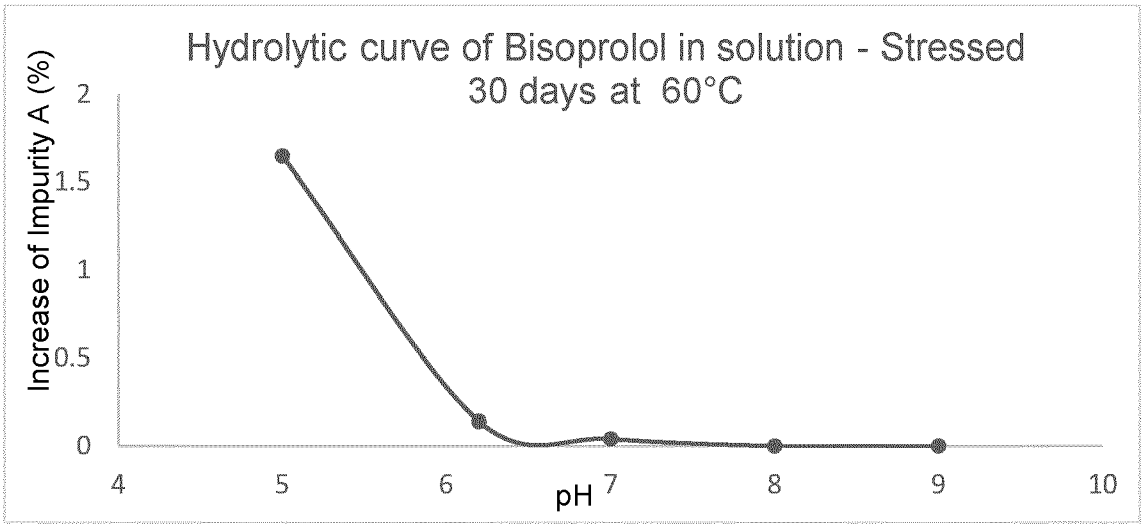
Hydrolytic curve of Bisoprolol in solution - Stressed
30 days at 60°C
Increase of Impurity A (%)
2
1.5
1
0.5
0
4
5
6
7
8
9
10
pH

Figure 1

Europäisches Patentamt
European Patent Office
Office européen des brevets

# EUROPEAN SEARCH REPORT

Application Number
EP 23 17 5439

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 1 650 849 A (SHANG BAOHU [CN]) 10 August 2005 (2005-08-10) * examples 1-3; claims 1-11 * ----- | 1-15,21 | INV. A61K9/00 A61K9/08 A61K31/138 |
| X | US 2009/264535 A1 (KANIKANTI VENKATA-RANGARAO [DE] ET AL) 22 October 2009 (2009-10-22) * examples 1-14 * ----- | 1-15,21 | |
| X | MATEUSZ SZALKA ET AL: "Kinetics of Hydrolysis of Bisoprolol Hemifumarate in Aqueous Acidic Solutions", INTERNATIONAL JOURNAL OF CHEMICAL KINETICS, WILEY, NEW YORK, NY, US, vol. 45, no. 11, 3 August 2013 (2013-08-03), pages 744-754, XP071657864, ISSN: 0538-8066, DOI: 10.1002/KIN.20809 * page 744, right column, paragraph 1 * ----- | 1-15,21 | |

| TECHNICAL FIELDS SEARCHED (IPC) |
|---|
| A61K |

The present search report has been drawn up for all claims

1

| Place of search | Date of completion of the search | Examiner |
|---|---|---|
| The Hague | 21 February 2024 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 17 5439

This annex lists the patent family members relating to the patent documents cited in the above-mentioned European search report. The members are as contained in the European Patent Office EDP file on
The European Patent Office is in no way liable for these particulars which are merely given for the purpose of information.

21-02-2024

| Patent document cited in search report | | Publication date | Patent family member(s) | | | Publication date |
|---|---|---|---|---|---|---|
| CN 1650849 | A | 10-08-2005 | NONE | | | |
| US 2009264535 | A1 | 22-10-2009 | AR | 060730 | A1 | 10-07-2008 |
| | | | AU | 2007245911 | A1 | 08-11-2007 |
| | | | BR | PI0711140 | A2 | 23-08-2011 |
| | | | CA | 2650786 | A1 | 08-11-2007 |
| | | | CN | 101431981 | A | 13-05-2009 |
| | | | CO | 6180495 | A2 | 19-07-2010 |
| | | | CR | 10407 | A | 30-03-2009 |
| | | | DE | 102006020604 | A1 | 08-11-2007 |
| | | | EC | SP088850 | A | 30-12-2008 |
| | | | EP | 2015728 | A2 | 21-01-2009 |
| | | | GT | 200800235 | A | 28-04-2010 |
| | | | JP | 2009535368 | A | 01-10-2009 |
| | | | KR | 20090014183 | A | 06-02-2009 |
| | | | PE | 20080149 | A1 | 06-04-2008 |
| | | | RU | 2008147216 | A | 10-06-2010 |
| | | | SV | 2008003080 | A | 26-11-2009 |
| | | | TW | 200808373 | A | 16-02-2008 |
| | | | US | 2009264535 | A1 | 22-10-2009 |
| | | | UY | 30315 | A1 | 30-11-2007 |
| | | | WO | 2007124869 | A2 | 08-11-2007 |
| | | | ZA | 200809269 | B | 30-12-2009 |

EPO FORM P0459

For more details about this annex : see Official Journal of the European Patent Office, No. 12/82